# EUROPEAN PATENT APPLICATION

(11) **EP 4 470 579 A1**
(43) Date of publication of application: **04.12.2024**
(21) Application number: 23305875.9
(22) Date of filing: 02.06.2023
(51) Int. Cl.: A61M 5/14, A61M 5/168, A61M 5/36, A61M 39/10, A61M 5/00, A61M 39/00

(54) **MEDICAL CONNECTION SYSTEM**

(71) Applicant: Guerbet, 93420 Villepinte (FR)
(72) Inventor: DAVAL, Jean, 69100 VILLEURBANNE (FR); BLAIN, Barbara, 69004 LYON (FR); ALLARD, Ludovic, 69390 MILLERY (FR)
(74) Representative: Regimbeau

(57) **Abstract**

A connection system for connecting a first medical line (101) to a second medical line (201) leading to an injector, comprising a first connector (100) coupled to the first medical line and a second connector (200) coupled to the second medical line, wherein the second connector (200) comprises a line path (210) for the second medical line (201), comprising an exposure section (220) for exposing the second medical line, and the connection system comprises a docking station (300) configured to receive the second connector (200) in a reception space so that the first connector (100) can be engaged with the second connector (200) by moving the first connector (100) in an engagement direction into the second connector (200) received in the reception space (304), and wherein the docking station (300) comprises an air sensor faced by the exposure section (220) when the second connector (200) is received into the reception space (304).

## Description

### TECHNICAL FIELD

The present invention generally relates to the field of medical fluid injection. More precisely, the invention is a connection system for connecting a first medical line to a second medical line leading to an injector, said connection system comprising a first connector coupled to the first medical line and a second connector coupled to the second medical line, the first connector configured to engage with the second connector to create a passageway for a medical fluid.

### BACKGROUND OF THE INVENTION

Injection devices are commonly used for injecting into a medical tubing a medical liquid from a medical liquid container. For example, the injection of medical liquid such as iodinated contrast agent is required in 70% of CT scan diagnosis cases. This injection, in about 70% of cases, is performed using an automated contrast agent injector and tubing is used to connect the automated injector to the patient.

The injector is kept for several years, and is used for many patients. On the opposite, the medical line on the patient's side leads to a skin penetration device such as a needle to penetrate the skin. This medical line is often referred to as the patient line. For obvious safety reasons, this patient line can only be used once for each unique patient, and needs to be changed between patients. There is a need for connecting the patient line to another medical line leading to the injector. This other medical line can be kept in place for several patients, usually for a whole day, and therefore is often called the day line or day set.

Because several patient lines are connected to and disconnected from the day line, it is beneficial and valuable for the connection between those lines to be easy and safe. As it is a routine operation, there is higher risk of human error due to inattention. To avoid injecting air bubbles in a patient, some systems are provided with an air sensor to detect the presence of air in the fluid to be injected. Most often, a medical line must be arranged manually in front of the air sensor, which can be burdensome and increases the risk or error.

Accordingly, there is a need for a connection system for connecting a first medical line to a second medical line leading to an injector which provides a safe and secure connection with a minimal requirement from the operator connecting the lines.

### SUMMARY OF THE INVENTION

It is proposed a connection system for connecting a first medical line to a second medical line leading to an injector, comprising a first connector coupled to the first medical line and a second connector coupled to the second medical line, the first connector configured to engage with the second connector to create a passageway for a medical fluid when the first connector is in an engaged configuration with the second connector, wherein the second connector comprises a line path for the second medical line, said line path comprising an exposure section for exposing the second medical line, and the connection system comprises a docking station configured to receive and hold the second connector in a reception space, and in that said first connector is configured to be engaged with the second connector into the engaged configuration by moving the first connector in an engagement direction into the second connector received in the reception space, and wherein the docking station comprises an air sensor, and the exposure section of the line path faces the air sensor when the second connector is received into the reception space.

The invention allows to connect the first medical line to the second medical line in a single move while positioning the second medical line in a proper position with respect to the air sensor.

Other preferred, although non-limitative, aspects of the invention are as follows, isolated or in a technically feasible combination:
- the first medical line is a patient tubular line leading to a skin penetration device and is configured to be changed between each patient, whereas the second medical line is a multi-use tubular line configured to be used with several patients,
- the line path comprises brackets configured to hold the second medical line in the line path, the exposure section being devoid of brackets,
- the line path comprises a first portion extending in the first direction, and a second portion extending in a second direction forming an angle with the first direction, wherein the exposure portion is arranged in the second portion,
- the air sensor comprises a groove configured to accommodate the second medical line at the exposure section of the line path when the second connector is in the reception space,
- the first connector comprises a resilient engagement member configured to cooperate with the second connector in the engaged configuration to lock the first connector with the second connector, and wherein the docking station comprises an optical detector configured to detect a locked state of the first connector with the second connector,
- the first connector comprises at least one resilient engagement member comprising an anchoring portion at a distal end, said resilient engagement member configured to have said distal end deflected to allow engagement of the first connector and the second connector, and to have the anchoring portion cooperating with a shoulder of the second connector to retain the first connector in a second direction opposite to the first direction once the first connector is engaged with the second connector,
- the docking station comprises an optical detector configured to emit a light beam and to detect whether the light beam is cut, wherein the light beam is located so as to be cut only by the first connector when the anchoring portion cooperates with the shoulder of the second connector, the system thus does not merely detect the presence of the first medical line, but detects the locked state of the first medical line, avoiding errors,
- the first connector comprises a resilient engagement member configured to cooperate with the second connector in the engaged configuration to lock the first connector with the second connector, said resilient engagement member comprising a distal end configured to be inserted into the second connector, and a first handling portion at a proximal end, wherein the first connector comprises two wings on two opposite sides of the first connector, and each wing comprises a second handling portion extending over an area larger than an area of the first handling portion,
- the proximal end of each resilient engagement member is separated from a body of the first connector by a clearance configured to accommodate part of the proximal end when the engagement member is deflected, and wherein said clearance is located between the two wings.

The invention also relates to a process for operating a connection system of the invention, comprising engaging the second connector in the reception space of the docking station, the second medical line being arranged along the line path of the second connector with the exposure section facing the air sensor, and then several successive times for different first connector and first medical line while the second connector stays in the reception space:
- engaging the first connector with the second connector by moving the first connector in an engagement direction into the second connector received in the reception space, said first connector coupled to the first medical line, thereby creating a passageway for a medical fluid between the second medical line, the second connector, the first connector and the first medical line,
- disengaging the first connector from the second connector by proximal end moving the first connector in a disengagement direction opposed to the engagement direction,
- discarding the first connector.

### BRIEF DESCRIPTION OF THE DRAWINGS

Other aspects, objects and advantages of the present invention will become better apparent upon reading the following detailed description of preferred embodiments thereof, given as non-limiting examples, and made with reference to the appended drawings wherein:
- Fig. 1 shows a global view of the connection system, with the second connector not yet received in the reception space of the docking station;
- Fig. 2a and 2b show a cross-sectional view of the second connector being received in the reception space of the docking station;
- Fig. 3a and 3b show another cross-sectional view of the second connector being received in the reception space of the docking station
- Fig. 4a, 4b, 4c, 4d show various steps of the engagement of the first connector with the second connector;
- Fig. 5 shows a variant of the first connector having additional wings for the insertion of the first connector,
- Fig. 6 shows another view of the first connector of Fig. 5.

### DETAILED DESCRIPTION OF THE INVENTION

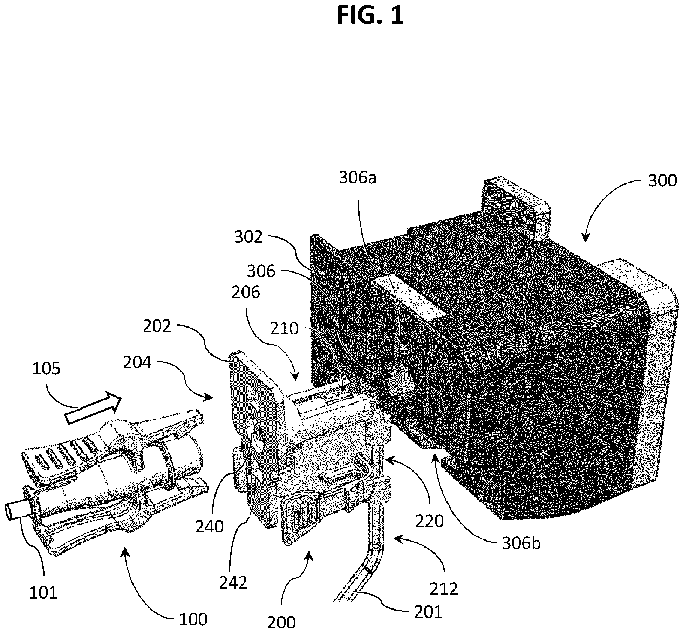

**Fig. 1** shows a global view of the connection system, comprising a first connector 100, a second connector 200 and a docking station 300. A fist medical line 101 is coupled to the first connector 100, and a second medical line 201 is connected to the second connector. The first medical line 101 is the patient line and leads to a skin penetration device such as a needle or a catheter, which allows a medical fluid to penetrate the patient's body. The second medical line 201 leads to an injector. The first medical line 101 is configured to be changed between each patient, whereas the second medical line 201 is a multi-use tubular line configured to be used with several patients.

The first connector 100 is configured to engage with the second connector 200 in an engaged configuration in order to create a passageway extending along the second medical line 201 and the first medical line 101 for a medical fluid to travel from the second medical line 201 to the first medical line 101. Such a medical fluid can then be injected by the injector through the second medical line 201, then through the first medical line 101, to finally reach the skin penetration device. The first connector 100 can be engaged with the second connector 200 by moving the first connector 100 in an engagement direction 105 into the second connector 200 when the second connector 200 is received by the docking station 300. The engagement direction 105 is the direction in which the first connector 100 is to be translated for passing from a disengaged configuration in which the first connector 100 is not engaged with the second connector 200, to an engaged configuration in which the first connector 100 is engaged with the second connector 200.

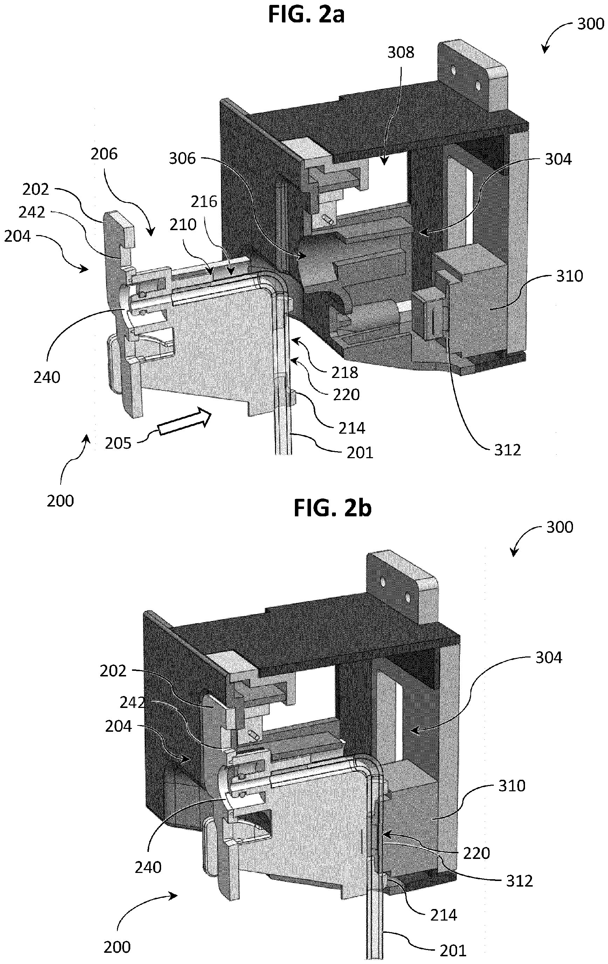

The docking station 300 comprises a face 302 in which a reception space 304 opens. The reception space 304 is configured to receive and hold the second connector 200 so that the first connector 100 can be engaged with the second connector 200. In the example of **Fig. 1** and **Fig. 2a****,** the second connector 200 is not yet engaged into the reception space 304. The docking station 300 is configured to affixed to or integrated into a fixed panel provided with electric power and communication connections or a processing unit.

The docking station 300 comprises an air sensor 310 facing the reception space 304. The air sensor 310 is configured to detect the presence of air in the second medical line 201. The air sensor 310 may be of any kind able to perform such a function. For example, the air sensor 310 may be an optical sensor or an ultrasound sensor, configured to detect gas or bubble in the medical fluid.

The second connector 200 comprises a face 202, in which opens an interface 204 for the engagement with the first connector 100, and a rear portion 206 which is configured to be received in the reception space 304. Preferably, the face 202 of the second connector 200 and the face 302 of the docking station are flush when the second connector 200 is arranged in the reception space 304.

The second connector 200 comprises a line path 210 for the second medical line 201. In the depicted example, the second medical line 201 is arranged along said line path 210. The line path 210 extends from the interface 204 to the rear portion 206, and comprises an exit 212 through which the second medical line 201 leaves the line path 210. The line path 210 may comprise brackets 214 configured to hold the second medical line 201 in the line path 210. Preferably, the line path 210 run on an outer surface of the rear portion 206 of the second connector 200.

As in the depicted example, the line path 210 may comprises a first portion 216 extending in a first direction, typically the engagement direction 105, and a second portion 218 extending in another, second direction, forming an angle with the first direction. Preferably, the second direction is substantially perpendicular (e.g. forming an angle between 80° and 100°) to the first direction in which the first portion 216 extends. The first portion 216 ends at the interface 204, and the second portion 218 prolongs the first portion 216 to the exit 212. The two portions of the line path 210 allow controlling the direction in which the second medical line 201 exits the line path 210, and prevent a pulling on the second medical line 201 to cause a decoupling of the medical line 201 from the second connector 200.

The line path 210 comprises an exposure section 220 for exposing the second medical line 201. At least at this exposure section 220, the second medical line 201 is exposed from the second connector 200, and the second medical line 201 is not covered by the second connector 200. Typically, the exposure section 220 is devoid of brackets used to hold the second medical line 201 on the line path 210. Preferably, the exposure portion 220 is arranged in the second portion 218, which extends in a second direction forming an angle with the first direction in which extends the first portion 216 of the path line 210, i.e., the engagement direction 105 in this example. The angle is preferably greater than 45°, and is preferably around 90°.

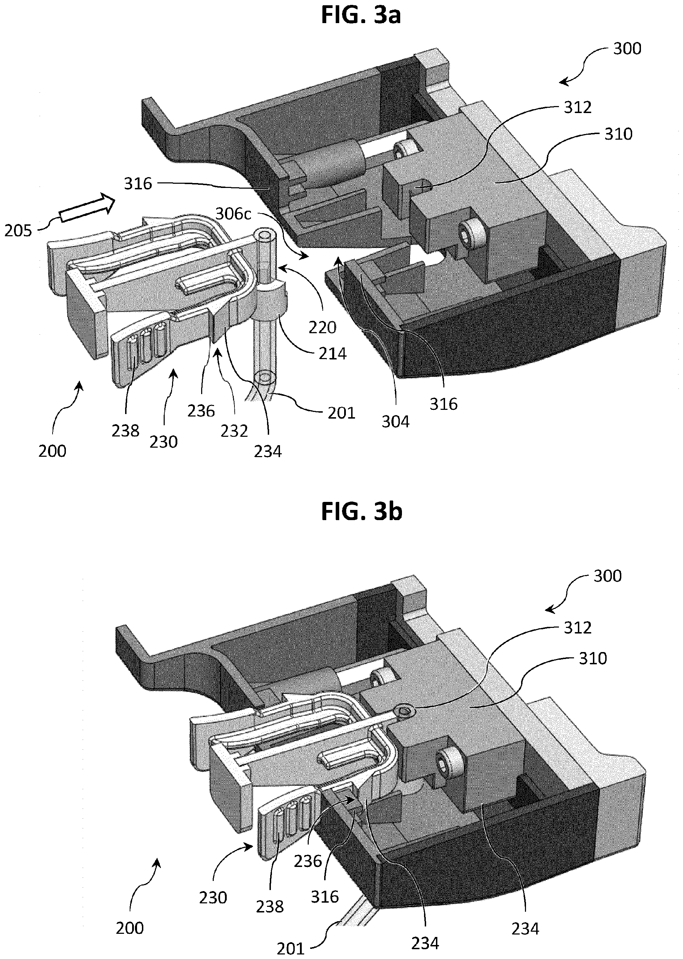

As depicted in **Fig. 2a, 2b****,** **3a, and 3b****,** the second connector 200 is inserted into the reception space 304 of the docking station by moving the second connector 200 along an insertion direction 205, which is a horizontal translation in the depicted example and which is shown by an arrow. In this example, the insertion direction 205 is the same as the engagement direction 105. It is however possible for the insertion direction 205 to be different from the engagement direction 105, and for example to be perpendicular from the engagement direction 105, typically with a vertical translation instead of a horizontal translation.

The reception space 304 opens in the face 302 of the docking station 300 and defines an orifice 306 in the face 302. The shape of the orifice 306 allows the insertion of the rear portion 206 of the second connector 200. The orifice 306 in the face 302 is preferably asymmetrical, thereby indicating how the second connector 200 must be oriented to be properly inserted into the reception space 304. In the depicted example, the orifice 306 comprises an upper slot 306a, and a round shape 306b below the upper slot 306a. An opening 306c prolongs the round shape 306b and reaches a lower face of the docking station 300.

The reception space 304 is located inside a cavity 308 within the docking station 300. The air sensor 310 is preferably arranged within the cavity 308, on a side of the reception space 304 opposed to the orifice 306 in the face 302 of the docking station 300. The air sensor 310 comprises a groove 312 configured to accommodate the second medical line 201 at the exposure section 220 of the line path 210 when the second connector 200 is in the reception space 304. The opening 306c extends underneath the air sensor 310, facing the exit 212 of the line path 210 when the second connector 200 is in the reception space 304. This allows the second medical line 201 to penetrate the reception space 304 and to reach the air sensor 310 while being held in the line path 210.

As illustrated, during the insertion of the second connector 200, the rear portion 206 penetrates the reception space 304 until the second medical line 201 positioned in the line path 210 reaches the air sensor 310 and penetrate the groove 312 of the air sensor 310. More precisely, the exposure section 220 of the line path 310 is received in the groove 312.

Once the second connector 200 is received in the reception space 304, the exposure section 220 of the line path 210 exposes the second medical line 201 to the air sensor 310. The air sensor 310 is then able to detect the presence of air in the second medical line 201. There is no need for any manual positioning of the second medical line 201 in front of the air sensor 310.

In order to ensure that the second connector 200 is kept in place, the second connector 200 comprises resilient engagement members 230 comprising an anchoring portion 232 projecting from the resilient engagement members 230. Upon insertion of the second connector 200, an inclined surface 234 of the anchoring portion 232 contacts a wall portion 316 of the docking station, which causes a deflection of the resilient engagement member 230 while the inclined surface 234 slides with respect to the front wall portion 316. The resilient engagement members 230 are forced into a compressed configuration. The inclined surface 234 is terminated by a retaining portion 236. When the wall portion 316 reaches the retaining portion 236, the resilient engagement members 230 spring back to a wider configuration. The retaining portions 236 then protrude behind the wall portion 316, and retain the second connector 200 in the reception space 304. The resilient engagement members 230 comprises gripping portion 238 at a distal end. Fingers of an operator can press those gripping portion 238 to deflect the resilient engagement members 230 to allow the second connector 200 to be disengaged from the docking station. It shall be noted that the operator preferably does not have to press the gripping portion 238 to insert the second connector 200 in the reception space 304. A mere translation of the second connector 200 in the insertion direction 205 is enough. To remove the second connector 200 from the reception space 304, the operator however has to press the gripping portion 238 to deflect the engagement members 230 and the retaining portions 236 out of engagement with the wall portion 316. The operator then translates the second connector 200 in a direction opposed to the insertion direction 205.

The connection system allows to combine actions in a single gesture: by moving the second connector 200 in the insertion direction 205 to be received and hold in the reception space 304 of the docking station 300, a connection interface is created for a single-use first connector 100 configured to lead to the patient line, i.e. the first medical line 101. Also, the second medical line is placed in front of the air sensor 310 at the exposure section 220 of the line path 210.

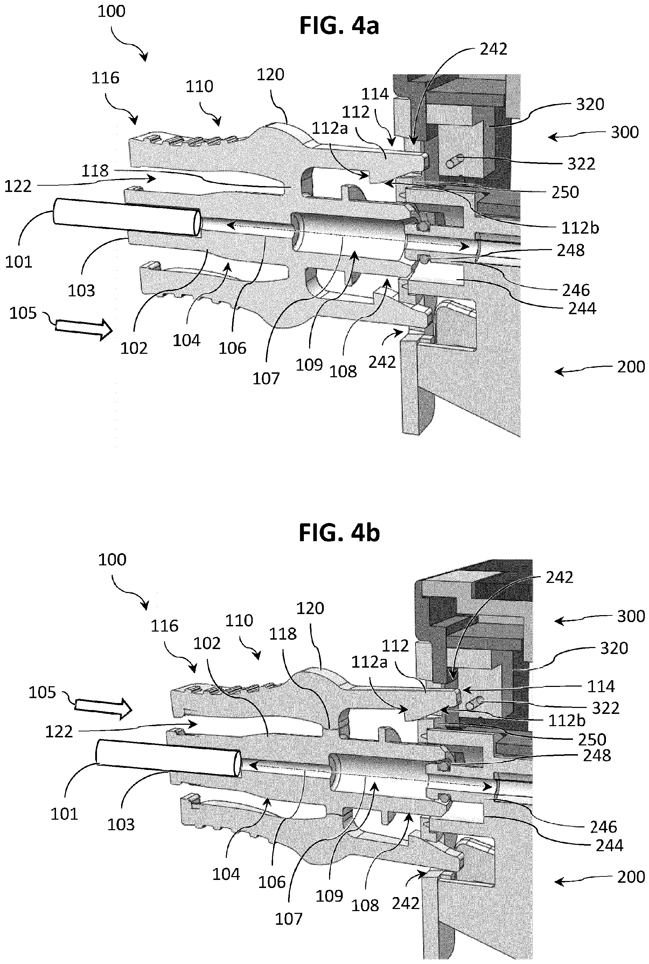

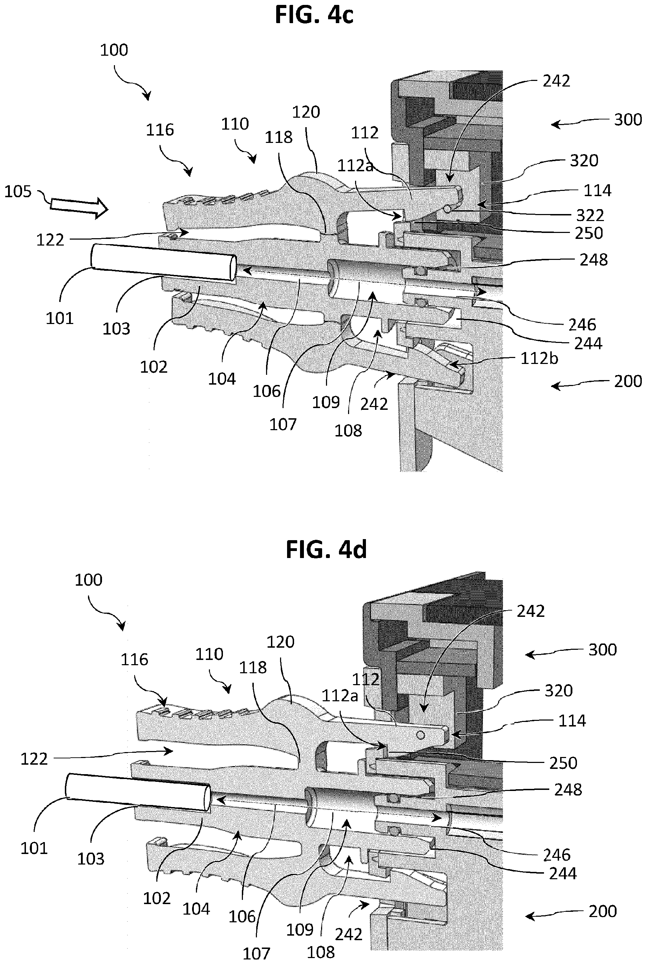

With reference to **Fig. 4a, 4b****,** **4c, and 4d****,** the engagement of the first connector 100 with the second connector 200 will now be described. As explained above, the second connector 200 is held in place by the docking station 300. The interface 204 in the face 202 of the connector is visible. The interface 204 comprises an aperture 240 and at least one window 242, preferably separated from the aperture 240. The aperture 240 comprises a groove 244 which is preferably circular, and surrounds a central conduit 246 provided with a toric joint or O-ring 248 on an outer surface of the conduit 246.

The first connector 100 comprises a body 102 comprising a sleeve 103 where the first medical line 101 is inserted, an intermediate section 104 comprising a channel 106 for leading the medical fluid out of the first medical line 101, and a head 108, in which the channel 106 opens. The head 108 is hollow to allow passage of the medical fluid therethrough. The interior 109 of the head 108 forms a part of the passageway 107. Preferably, the head 108 has a circular cross-section, and the interior 109 of the head 108 also has a circular cross-section.

In order to engage the first connector 100 with the second connector 200, the head 108 of the first connector 100 is placed to face the aperture 242 of the second connector 200, and then is translated in the engagement direction 105.

The first connector 100 comprises at least one resilient engagement member 110 comprising a distal end 114 configured to be inserted into the second connector 200 and a proximal end 116 configured to be left outside the second connector 200 in the engaged configuration. The engagement member 110 comprises an anchoring portion 112 at a distal end 114, and a first handling portion 115 at a proximal end 116. Typically, the first connector comprises two resilient engagement members 110, preferably arranged on opposite sides of the head 108.

Preferably, the resilient engagement member 110 is connected to the body 102 of the first connector 100 by an arm 118 extending from a middle section 120 of the resilient engagement member 110 to join the body 102, e.g. the head 108 or the intermediate section 104 of the first connector 100. The arm 118 acts as a fulcrum about which the engagement member 110 pivots between a first, resting position and a second position in which the distal end 114 is spread from the body 102 of the first connector 100. The second position is required to allow engagement of the first connector 100 and the second connector 200.

The proximal end 116 of each resilient engagement member 110 is separated from the body 102 of the first connector 100 by a clearance 122 in which the proximal end 116 is configured to penetrate when the resilient engagement member 110 is deflected in the second position, e.g. pivots about the arm 118.

For connecting the first connector 100 to the second connector 200, the first connector 100 is brought in front of the interface 204 of the second connector 200, so that the head 108 of the first connector 100 faces the aperture 242 of the second connector 200.

The first connector 100 is then engaged with the second connector 200 by moving the first connector 200 in the engagement direction 105 into the second connector 200 received in the reception space 304. The head 108 penetrates the groove 244 of the interface 204, while the conduit 246 enters the interior 109 of the head 108. In this example, the engagement direction 205 is thus opposed to the direction in which the conduit 246 protrudes from the groove 244. Once the first connector 100 is engaged with the second connector 200, the passageway 107 leading from the second medical line 201 to the first medical line 101 extends through the central conduit 246 of the second connector 200, the interior 109 of the head 108, and channel 106 of the intermediate section 104.

The distal end 114 of each resilient engagement member 110 penetrates a window 242. An inclined surface 112a of the anchoring portion 112 contacts an edge of the window 242, which causes a deflection of the resilient engagement member 110 while the inclined surface 112a slides with respect to the edge of the window 242. The resilient engagement member 110 rotates around the arm 118, and the distal end 114 moves away from the head 108 while the proximal end 116 gets closer to the sleeve 103 (See **Fig. 4a, 4b****, and** **4c**).

The anchoring portion 112 comprises a retaining portion 112b terminating the inclined surface 112a. Once the sliding between the inclined surface 112a and the edge of the window 242 reaches the end of the inclined surface 112a, the edge of the window 242 faces a recess formed by the retaining portion 112b. This allows the resilient engagement member 110 to spring back towards its initial configuration. The resilient engagement member 110 rotates around the arm 118, and the distal end 114 gets closer to the head 108 while the proximal end 116 moves away from the sleeve 103. The retaining portion 112b protrudes behind the edge of the window 242, which forms a shoulder 250 on which abuts the retaining portion 112b. The first connector 100 is thus retained in engagement with the second connector 200. The first connector 100 is thus locked to the second connector 200, as illustrated on **Fig. 4d****,** preventing the first connector 100 from being removed from the engaged configuration by a traction on the first connector 100.

The docking station 300 comprises an optical detector 320 configured to detect a locked state of the first connector 100 on the second connector 200. The optical detector 320 configured to emit a light beam 322, such as a laser beam or an infrared beam. The optical detector 320 is configured to detect whether the light beam 322 is cut. Typically, the optical detector 320 comprises a light detector on which the light beam 322 impinges when not cut, it is considered that the light beam 322 is cut when the light detector does not detect the light beam 322.

The light beam 322 of the optical detector 320 extends behind the window 242 in the engagement direction 105. More precisely, the light beam 322 is located so as to be cut only by the first connector 200 when the anchoring portion 112 cooperates with the shoulder 250 of the second connector 200. As illustrated particularly on **Fig. 4c****,** as long as the inclined surface 112a is in contact with the edge of the window 242, the light beam 322 is not cut by the distal end 114 of the first connector 100. In this way, the optical detector 320 does not merely detect the presence of the first connector 100 in its engaged configuration, but detects the locked state of the first connector 100, thus avoiding any error and allowing to confirm that the first connector 100 is properly locked in the engaged configuration.

The detection of the locked state of the first connector 100 by the optical detector 320 may be transmitted to a communication device from which the operator can be informed of the locked state detection. For example, the communication device can be a mere light source, a display screen, a loudspeaker, etc., that allows the operator to know whether the first connector 100 is properly locked with the second connector 200.

The connection system allows to simplify the creation of a passageway 107 for a medical fluid between the second medical line 201, the second connector 200, the first connector 100 and the first medical line 201, since it requires only a single gesture of engaging the first connector 100 with the second connector 200 by translating the first connector 100 in the engagement direction, while assuring that the medical fluid passes in front of the air sensor 310 at the exposure section 220 of the line path 210.

The first connector 100 can be disengaged from the second connector 200 by moving the first connector 100 in a disengagement direction opposed to the engagement direction 105. The first connector 100 however must be unlocked. Fingers of an operator press the first handling portion 115 at the proximal end 116 of the resilient engagement member 110 to deflect it in order to unlock the first connector 100 and allow a translation of the first connector in the disengagement direction opposed to the engagement direction 105, thus disengaging the first connector 100 from the second connector 200. The first handling portion 115 extends at a distance from the intermediate section 104 to allow the first handling portion 115 to be deflected towards said intermediate section 104 and the sleeve 103. The first handling portion is configured to be pressed by a finger of an operator to deflect the engagement member 110, thereby spreading the distal end 114 of the engagement member 110 out of engagement with the shoulder 250 of the second connector 200. Once the distal end 114 is disengaged from the shoulder 250 and faces the window 242, the first connector 100 is translated in a disengagement direction opposed to the engagement direction 105. The first connector 100 and the second connector 200 are no longer coupled.

The first connector 100 is then discarded, typically thrown away. Another first connector 100 for another patient may then be connected to the second connector 200. This process can be performed several successive times for different first connector 100 and first medical line 201 while the second connector 200 stays in the reception space 304.

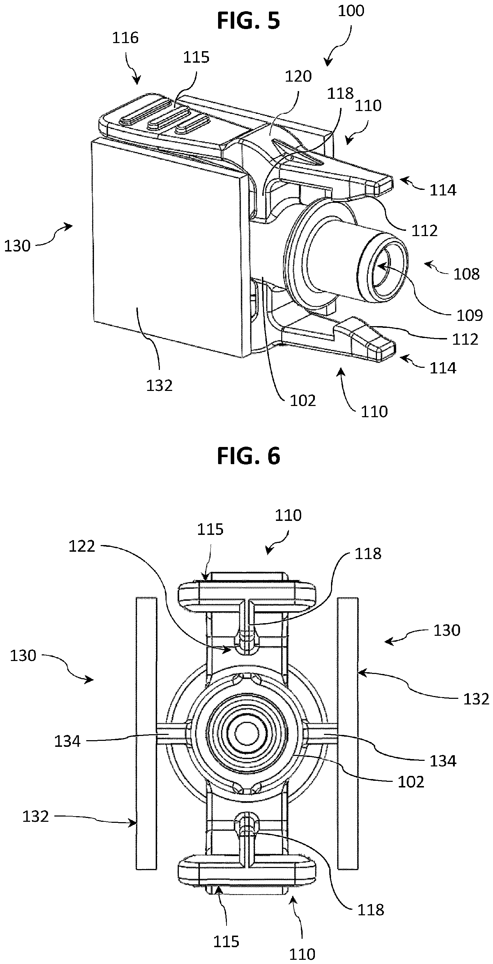

As shown on **Fig. 5** and **Fig. 6****,** in order to allow an easier handling of the first connector 100 when moving the first connector 100 in the engagement direction 105 for coupling it with the second connector 200, the first connector 100 may be provided with two additional wings 130 on either side of the sleeve 103 and/or intermediate section 104. Each additional wing 130 comprises a second handling portion 132 configured to be pressed by a finger of the operator to hold the first connector 100 when engaging it into the second connector 200. Preferably, each wing 130 is connected to the sleeve 103 and/or intermediate section 104 by a strut 134.

The two wings 130 cover two lateral sides of the first connector 100 when the first connector 100 is positioned to be inserted into the second connector 200, and preferably extend at least from the intermediate section 104 to the sleeve 103. Preferably, when the first connector 100 is arranged to be inserted into the second connector 200 or is actually coupled to the second connector 200, the wings 130 are located on the sides of the first connector 100, and the second handling portions 132 extend from the top to the bottom of the first connector 100, whereas at least a first engagement member 110 is at the top of the first connector 100. Preferably, a second engagement member 110 is at the bottom of the first connector 100.

Each second handling portion 132 extends over an area which is preferably larger than the area of the first handling portion 115 of the engagement member 110, more preferably at least three times larger. Intuitively, upon removal of the connection system or of the first connect 100 from the package, the operator will want to take the larger shape and thus grasp the wings 130 between their thumb and index finger. By doing so, the first handling portion 115 of the engagement member 110 is not pressed.

As fingers of the operators do not press the first handling portion 115, the engagement member 110 is not deflected except when the inclined surface 112a is in contact with the edge of the window 242, fingers do not prevent the engagement member 110 from springing back once the sliding between the inclined surface 112a and the edge of the window 242 reaches the end of the inclined surface 112a. This causes a sensory feedback for the operator, for example an haptic feedback and/or a noise such a click, indicating to the operator that the first connector 100 is secured to the second connector 200.

Further, by placing the fingers on the wings 130, the distal end 114 of at least one resilient engagement member 110 is still visible and the operator is able to guide said visible distal end 114 into the window 242 of the interface 204 of the second connector 200.

Preferably, the wings 130 are interposed between the proximal ends 116 of the two engagement members 110. Preferably, the wings 130 extend at least at the level of the first handling portion 115 of an engagement member 110. The clearance 122 between the proximal end 116 of the engagement member 110 and the body 102 of the first connector 100, configured to accommodate part of the proximal end 116 when the engagement member 110 is deflected, is located between the two wings 130. Accordingly, pressing the first handling portion 115 requires inserting the pressing finger between the two wings 130, thereby preventing any accidental pressing of the first handling portion 115 which would result in an undesired disengagement of the first connector 100 from the second connector 200.

While the present invention has been described with respect to certain preferred embodiments, it is obvious that it is in no way limited thereto and it comprises all the technical equivalents of the means described and their combinations. In particular, it will be apparent to those skilled in the art that various changes and modifications may be made without departing from the scope of the invention as defined in the appended claims.

## Claims

1. A connection system for connecting a first medical line (101) to a second medical line (201) leading to an injector, comprising a first connector (100) coupled to the first medical line (101) and a second connector (200) coupled to the second medical line (201), the first connector (100) configured to engage with the second connector (200) to create a passageway (107) for a medical fluid when the first connector (100) is in an engaged configuration with the second connector (200),
**characterized in that** the second connector (200) comprises a line path (210) for the second medical line (201), said line path (210) comprising an exposure section (220) for exposing the second medical line, and
**in that** the connection system comprises a docking station (300) configured to receive and hold the second connector (200) in a reception space (304), and **in that** said first connector (100) is configured to be engaged with the second connector (200) into the engaged configuration by moving the first connector (100) in an engagement direction (105) into the second connector (200) received in the reception space (304), and wherein the docking station (300) comprises an air sensor (310), and the exposure section (220) of the line path (210) faces the air sensor (310) when the second connector (200) is received into the reception space (304).

2. The connection system of claim 1, wherein the first medical line (101) is a patient tubular line leading to a skin penetration device and is configured to be changed between each patient, whereas the second medical line (201) is a multi-use tubular line configured to be used with several patients.

3. The connection system of any one of claims 1 to 2, wherein the line path (210) comprises brackets (214) configured to hold the second medical line (201) in the line path (210), the exposure section (220) being devoid of brackets.

4. The connection system of any one of claims 1 to 3, wherein the line path (210) comprises a first portion (216) extending in the first direction, and a second portion (218) extending in a second direction forming an angle with the first direction, wherein the exposure portion (220) is arranged in the second portion (218).

5. The connection system of any one of claims 1 to 4, wherein the air sensor (310) comprises a groove (312) configured to accommodate the second medical line (201) at the exposure section (220) of the line path (201) when the second connector (200) is in the reception space (304).

6. The connection system of any one of claims 1 to 5, wherein the first connector comprises a resilient engagement member (110) configured to cooperate with the second connector (200) in the engaged configuration to lock the first connector (100) with the second connector (200), and wherein the docking station comprises an optical detector (320) configured to detect a locked state of the first connector (100) with the second connector (200).

7. The connection system of any one of claims 1 to 6, wherein the first connector (100) comprises at least one resilient engagement member (110) comprising an anchoring portion (112) at a distal end (114), said resilient engagement member (110) configured to have said distal end (114) deflected to allow engagement of the first connector (100) and the second connector (200), and to have the anchoring portion (112) cooperating with a shoulder (250) of the second connector (200) to retain the first connector (100) in a second direction opposite to the first direction once the first connector (100) is engaged with the second connector (200).

8. The connection system of claim 7, wherein the docking station comprises an optical detector (320) configured to emit a light beam (322) and to detect whether the light beam (322) is cut, wherein the light beam (322) is located so as to be cut only by the first connector (100) when the anchoring portion (112) cooperates with the shoulder (250) of the second connector (200).

9. The connection system of any one of claims 1 to 8, wherein the first connector comprises a resilient engagement member (110) configured to cooperate with the second connector (200) in the engaged configuration to lock the first connector (100) with the second connector (200), said resilient engagement member (110) comprising a distal end (114) configured to be inserted into the second connector (200), and a first handling portion (115) at a proximal end (116), wherein the first connector (100) comprises two wings (130) on two opposite sides of the first connector (100), and each wing (130) comprises a second handling portion (132) extending over an area larger than an area of the first handling portion (115).

10. The connection system of claim 9, wherein the proximal end (116) of each resilient engagement member (110) is separated from a body (102) of the first connector (100) by a clearance (122) configured to accommodate part of the proximal end (116) when the engagement member (110) is deflected, and wherein said clearance (122) is located between the two wings (130).

11. A process for operating a connection system according to any one of claims 1 to 10, comprising engaging the second connector (200) in the reception space (304) of the docking station (300), the second medical line (201) being arranged along the line path (210) of the second connector (200) with the exposure section (220) facing the air sensor (310), and then several successive times for different first connector (100) and first medical line (101) while the second connector (200) stays in the reception space (304):
- engaging the first connector (100) with the second connector (200) by moving the first connector (100) in an engagement direction (105) into the second connector (200) received in the reception space (304), said first connector (100) coupled to the first medical line (201), thereby creating a passageway (107) for a medical fluid between the second medical line (201), the second connector (200), the first connector (100) and the first medical line (201),
- disengaging the first connector (100) from the second connector (200) by proximal end (116) moving the first connector (100) in a disengagement direction opposed to the engagement direction,
- discarding the first connector (100).

12. The process of claim 11, wherein the first connector comprises a resilient engagement member (110) configured to cooperate with the second connector (200) in the engaged configuration to lock the first connector (100) with the second connector (200), said resilient engagement member (110) comprising a distal end (114) configured to be inserted into the second connector (200), and a first handling portion (115) at a proximal end (116), wherein the first connector (100) comprises two wings (130) on two opposite sides of the first connector (100), wherein:
- engaging the first connector (100) with the second connector (200) comprises holding the first connector (100) with fingers on the wings (130), and
- disengaging the first connector (100) from the second connector (200) comprises pressing the first handling portion (115) at a proximal end (116) of the engagement member (100) when moving the first connector (100) in a disengagement direction opposed to the engagement direction (105).
